# FASCICULE DE BREVET EUROPEEN

(11) **EP 4 136 426 B1**
(45) Date de publication et mention de la délivrance du brevet: **01.04.2026**
(21) Numéro de dépôt: 21718121.3
(22) Date de dépôt: 13.04.2021
(51) Int. Cl.: G01N 1/20, B01D 21/26, G01N 1/22, G01N 15/02, G01N 15/04, G01N 33/28

(54) **SURVEILLANCE DE L'HUILE D'UN DISPOSITIF DE LUBRIFICATION**
ÜBERWACHUNG DES ÖLS EINER SCHMIERVORRICHTUNG
MONITORING THE OIL OF A LUBRICATION DEVICE

(30) Priorité: 14.04.2020 BE 202005244
(43) Date de publication de la demande: 22.02.2023
(73) Titulaire: Safran Aero Boosters SA, 4041 Herstal (BE)
(72) Inventeur: RAIMARCKERS, Nicolas, Oscar, Louis, Ghislain, 4041 Herstal (BE); BOUGELET, Stéphane, Alain, Luc, Ghislain, 4041 Herstal (BE)
(74) Mandataire: Ipsilon Luxembourg
(86) Numéro de dépôt international: PCT/EP2021/059575
(87) Numéro de publication internationale: WO 2021/209456

(56) Documents cités:
- US-A- 5 028 318
- US-A1- 2009 183 950
- US-A1- 2017 056 894
- US-B1- 6 348 087
- US-B1- 7 288 139
- US-B2- 10 434 523

## Description

### Domaine technique

L'invention a trait au domaine de la lubrification, plus particulièrement de la lubrification dans une turbomachine (notamment d'aéronef), plus particulièrement encore à la surveillance de l'huile de lubrification.

### Technique antérieure

Le document de brevet publié EP 3 150 265 A1 divulgue un réservoir d'huile de turbomachine, équipé à l'entrée d'un désaérateur rotatif. L'huile chargée d'air arrive latéralement à une entrée située à une partie haute du réservoir. Cette entrée débouche dans une cavité logeant un rotor configuré pour être entrainé par le flux d'huile chargée d'air. La rotation du rotor projette les particules d'huile sur une paroi latérale de la cavité, celles-ci s'écoulant alors par gravité vers le réservoir. L'air ainsi séparé des particules d'huile est évacué vers un évent situé au-dessus du rotor.

Le document de brevet publié FR 2 443 691 A1 divulgue un détecteur de présence de particules magnétisables dans de l'huile, basé sur une mesure de résistance électrique. Un aimant permanent est disposé dans le détecteur de manière à attirer et accumuler les particules ferromagnétiques contenues dans l'huile et circulant à proximité du détecteur. Cette accumulation de particules ferromagnétiques forme un pont électriquement conducteur modifiant la résistante électrique mesurée. Ce détecteur est destiné à être monté au travers d'une paroi inférieure d'un carter d'huile.

Le document de brevet publié WO 2007/088015 A1 divulgue un détecteur de particules ferromagnétiques dans un débit d'huile circulant dans un tuyau. Le principe de détection est basé sur le magnétisme avec une bobine émettrice et une bonne réceptrice, et ne peut fonctionner qu'avec des particules ferromagnétiques. Le débit d'huile potentiellement important rend cependant la détection délicate et potentiellement incertaine.

Le document de brevet publié EP 3 220 168 A1 divulgue un détecteur de particules ferromagnétiques dans de l'huile de lubrification d'une turbomachine, basé sur le magnétisme avec une bobine émettrice et une bobine réceptrice, similairement à l'enseignement précédent (WO 2007/088015 A1). Le détecteur est prévu pour être disposé latéralement à une conduite dans laquelle l'huile de lubrification circule. Le détecteur comprend un aimant permanent pour attirer les particules ferromagnétiques, leur accumulation modifiant le champ magnétique mesuré par la bobine réceptrice.

Ces différents détecteurs présentent l'inconvénient de ne détecter que les particules ferromagnétiques et peuvent manquer de détecter certaines particules, en raison d'un manque d'accumulation de ces particules par l'aimant permanent ou par une taille et/ou une concentration trop faible pour être détectée (WO 2007/088015 A1).

### Résumé de l'invention

### Problème technique

L'invention a pour objectif de pallier au moins un des inconvénients de l'état de la technique susmentionné. Plus particulièrement, l'invention a pour objectif d'améliorer la détection de particules dans l'huile de lubrification d'une turbomachine.

### Solution technique

L'invention a pour objet un dispositif de détection de particules dans une huile de lubrification d'une turbomachine, comprenant un séparateur des particules ; au moins un détecteur des particules ; remarquable en ce que ledit dispositif comprend, en outre, un conduit de dérivation pour l'huile concentrant les particules, relié de manière fluidique à une sortie d'huile du séparateur des particules,; et en ce que l'au moins un détecteur de particules est monté de manière opérationnelle sur le conduit de dérivation de manière à pouvoir détecter les particules dans ledit conduit de dérivation.

Un support soutenant mécaniquement le conduit de dérivation peut être prévu. Il peut être rigidement lié au séparateur des particules.

Selon un mode avantageux de l'invention, le séparateur des particules comprend un bassin de décantation de l'huile, la sortie d'huile étant une sortie fluidique dudit bassin de décantation.

Selon un mode avantageux de l'invention, le séparateur des particules comprend une paroi de ruissellement pour l'huile vers le bassin de décantation.

Selon un mode avantageux de l'invention, la paroi de ruissellement est circulaire et forme un cyclone pour un mélange de l'huile avec de l'air.

Selon un mode avantageux de l'invention, le séparateur de particules est formé dans un séparateur air/huile.

Selon un mode avantageux de l'invention, le séparateur air/huile est du type cyclonique avec une entrée pour l'huile chargée d'air, une sortie d'air et une sortie d'huile déchargée d'air, le bassin de décantation étant situé de manière fluidique entre l'entrée pour l'huile chargée d'air et la sortie d'huile déchargée d'air.

Selon un mode avantageux de l'invention, l'au moins un détecteur de particules comprend un détecteur optique apte à détecter des particules non ferromagnétiques.

Selon un mode avantageux de l'invention, l'au moins un détecteur de particules comprend au moins un détecteur magnétique apte à détecter des particules ferromagnétiques.

Selon un mode avantageux de l'invention, le conduit de dérivation est un premier conduit de dérivation et la sortie d'huile du séparateur de particules est une première sortie d'huile, ledit dispositif détection comprenant au moins un deuxième conduit de dérivation relié de manière fluidique à une deuxième sortie d'huile du séparateur des particules, concentrant les particules, et au moins un de l'au moins un détecteur de particules est monté de manière opérationnelle sur le deuxième conduit de dérivation de manière à pouvoir détecter les particules dans ledit conduit de dérivation.

Selon un mode avantageux de l'invention, le bassin de décantation de l'huile est un premier bassin de décantation, le séparateur des particules comprenant un deuxième bassin de décantation de l'huile, la deuxième sortie d'huile étant une sortie fluidique dudit deuxième bassin de décantation.

Avantageusement, le ou les conduits de dérivation présentent, chacun, une section moyenne inférieure ou égale à 700mm², préférentiellement 600mm², plus préférentiellement 500mm².

Avantageusement, le dispositif détection est configuré pour que l'écoulement d'huile dans le ou chacun des conduits de dérivation présente une vitesse inférieure ou égale à 2m/s, préférentiellement 1m/s, plus préférentiellement 0.5m/s.

Avantageusement, le conduit de dérivation est distinct du séparateur des particules. Avantageusement, le conduit de dérivation est externe au séparateur des particules. Avantageusement, le conduit de dérivation comprend une sortie fluidique distincte du séparateur des particules de manière à pouvoir être connectée à un écoulement principal depuis le séparateur de particules vers une enceinte, ou directement à ladite enceinte. Avantageusement, le conduit de dérivation forme une boucle en U.

L'invention a également pour objet un réservoir d'huile de lubrification pour système de lubrification d'une turbomachine, notamment d'aéronef, comprenant : une enceinte pour l'huile de lubrification ; un dispositif de détection de particules dans l'huile de lubrification, disposé en amont de l'enceinte pour l'huile de lubrification ; remarquable en ce que le dispositif de détection est selon l'invention.

Selon un mode avantageux de l'invention, le séparateur des particules est à distance de l'enceinte, un conduit reliant de manière fluidique ledit séparateur de particules à la dite enceinte.

Selon un mode avantageux de l'invention, le dispositif de détection est rigidement fixé à l'enceinte par un support.

Selon un mode avantageux de l'invention, le séparateur des particules est intégré dans l'enceinte.

Avantageusement, le conduit de dérivation rejoint l'écoulement d'huile principal depuis le séparateur de particules vers l'enceinte, ou directement l'enceinte.

Avantageusement, le dispositif de détection de particules est situé à une partie haute de l'enceinte, préférentiellement au-dessus de l'enceinte.

L'invention a également pour objet un système de lubrification de turbomachine, notamment d'aéronef, comprenant des conduites d'amenée et de retour d'huile de lubrification ; au moins une pompe de circulation de l'huile de lubrification dans les conduites ; un réservoir d'huile de lubrification relié de manière fluidique aux conduites et à l'au moins une pompe ; remarquable en ce que le réservoir d'huile de lubrification est selon l'invention.

L'invention a également pour objet une turbomachine, notamment d'aéronef, comprenant un dispositif de détection de particules dans une huile de lubrification, caractérisé en ce que ledit dispositif de détection est selon l'invention.

L'invention a également pour objet une turbomachine comprenant un réservoir d'huile de lubrification pour système de lubrification, caractérisé en ce que ledit réservoir d'huile de lubrification est selon l'invention.

L'invention a également pour objet une turbomachine comprenant un système de lubrification, caractérisé en ce que ledit système de lubrification est selon l'invention.

### Avantages de l'invention

Les mesures de l'invention sont intéressantes en ce qu'elles permettent de réaliser une meilleure détection de particules dans une huile de lubrification. La détection est meilleure en ce que les particules sont détectées de manière plus fiable indépendamment de leur morphologie.

La morphologie des particules peut en effet avoir un impact important sur leur détection. La morphologie des particules peut être caractérisée par le rapport masse/surface, directement dépendant du diamètre moyen dans le cas de particules proches d'une forme sphérique ou d'un rapport entre la plus grande dimension et la plus petite dimension dans le cas de particules de forme non-sphérique, et aussi de la masse volumique de leur matériau.

La qualité de détection dépend de la ségrégation des particules au niveau du séparateur de particules et également de la qualité de leur détection dans la conduite de dérivation. Le fait de prévoir un conduit de dérivation à la sortie du séparateur de particules permet à ce dernier de se vider progressivement et ainsi d'éviter une accumulation et saturation. Le débit dans le conduit de dérivation permet un transport stabilisé et contrôlé des particules avec une concentration sensiblement plus importante que dans le débit principal en raison d'un débit plus faible. Une telle approche permet de s'acquitter des difficultés de détection de particules de morphologies particulières, comme notamment avec des rapports masse/surface faibles.

Le conduit de dérivation est aussi intéressant en ce qu'il permet de prévoir plusieurs détecteurs de particules en série le long du conduit en question. Cela signifie que la ségrégation des particules par le séparateur de particules et leur déplacement à vitesse contrôlée et à plus haute concentration sont mis à profit pour ces plusieurs détecteurs de particules.

Le fait de pouvoir prévoir plusieurs détecteurs de particules permet à faible surcoût de détecter différents matériaux, comme notamment les matériaux non-ferromagnétiques et les matériaux non-métalliques. Il est en effet maintenant courant de prévoir des roulements ou paliers en matériau céramique, susceptibles de produire de particules en matériau céramique.

### Brève description des dessins

La figure 1 est une vue schématique en couple longitudinale d'une turbomachine, illustrant le système de lubrification de la turbomachine.
La figure 2 est une représentation hydraulique de la partie réservoir du système de lubrification de la figure 1, détaillant un dispositif de détection de particules dans une huile de lubrification selon l'invention.
La figure 3 est une vue en perspective d'un séparateur air/huile avec un dispositif de détection de particules, tel que schématisé à la figure 2.
La figure 4 est une vue en coupe d'un séparateur air/huile avec un dispositif de détection de particules, tel que schématisé à la figure 2, intégré dans un réservoir d'huile de lubrification de turbomachine.

### Description d'un mode de réalisation

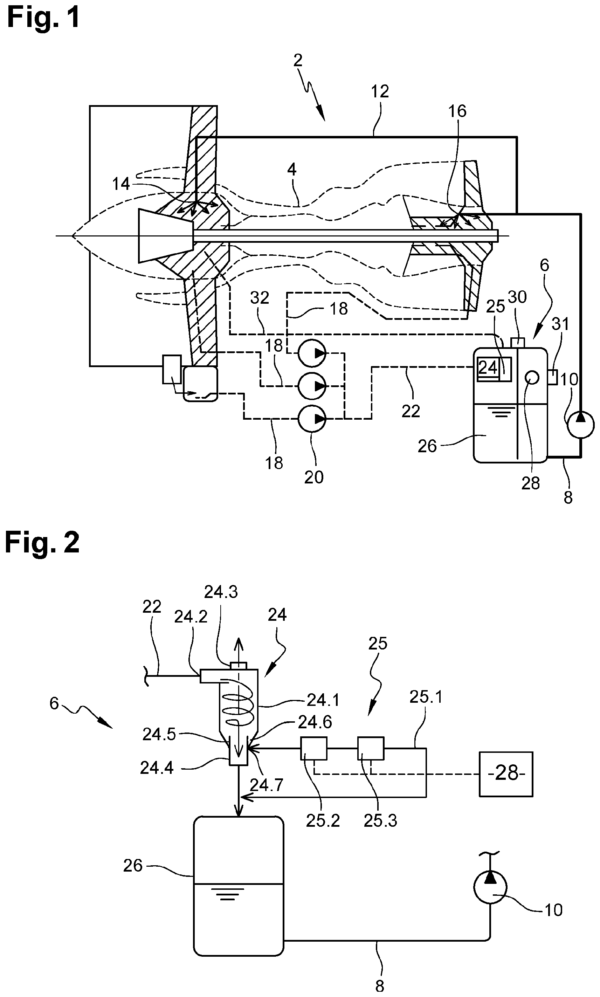

A la figure 1 est illustré un système de lubrification d'un moteur d'aéronef 4. Le système de lubrification 2 comprend essentiellement un réservoir d'huile 6, une conduite de sortie 8 reliée à une pompe d'alimentation 10. Des conduites 12 acheminent l'huile déplacée par la pompe de lubrification vers différentes enceintes de paliers à lubrifier 14 et 16 aux parties avant et arrière du moteur 4. L'huile est ensuite récupérée au fond de ces enceintes par des conduites 18 de récupération ainsi que par une ou des pompes de récupération 20. Cette huile chargée d'air est ensuite réacheminée via la conduite 22 vers le réservoir 6. Ce dernier comprend une enceinte 26 avec un séparateur air/huile 24 disposé à une partie haute de l'enceinte 26 et connecté à la conduite de retour d'huile 22. Le réservoir 6 peut comprendre également une fenêtre de contrôle visuelle 28 du niveau normal, d'un détecteur de niveau 30 ainsi qu'un détecteur de niveau complémentaire 31. La partie haute de l'enceinte 26 du réservoir 6 est également reliée via une conduite 32 à une ou plusieurs enceintes 14 et 16 du moteur, et ce, afin de permettre l'évacuation de l'air des pompes de récupération, cet air étant alors séparé de l'huile.

Les pompes d'alimentation et de récupération 10 et 20 sont préférentiellement du type volumétrique et entrainées par l'arbre principal du moteur. Lorsque le moteur est arrêté, l'huile présente dans les enceintes de lubrification et les conduites d'alimentation et de récupération revient vers le réservoir 6.

Le séparateur air/huile 24 est couplé à un dispositif de détection 25 de particules dans l'huile.

La figure 2 détaille la partie réservoir du circuit de lubrification de la figure 1, en particulier le dispositif 25 de détection de particules dans l'huile.

Le séparateur air/huile 24 est en l'occurrence du type cyclonique, à savoir configuré pour former un cyclone avec le débit d'huile chargée d'air en vue de projeter les particules d'huile contre une paroi circulaire et de guider et séparer l'air ainsi déchargé des particules d'huile. Plus particulièrement au séparateur air/huile 24 de la figure 2, ce dernier comprend une paroi 24.1 circulaire fermée, en l'occurrence généralement cylindrique, avec une entrée latérale 24.2 pour le débit d'huile chargée d'air acheminée par la conduite 22, une sortie, en l'occurrence centrale, d'air séparé de l'huile 24.3 et une sortie, en l'occurrence centrale, d'huile séparée de l'air 24.4. Les sorties d'air 24.3 et d'huile 24.4 sont opposées suivant l'axe longitudinal de la paroi circulaire 24.1. La sortie d'huile 24.4 est située à un niveau bas afin de pouvoir récolter par gravité l'huile séparée de l'air, celle-ci s'écoulant le long de la paroi circulaire 24.1. La sortie d'air 24.3 est située à un niveau haut opposé.

La paroi circulaire 24.1 présente avantageusement un profil conique à une partie basse adjacente à la sortie centrale d'huile 24.4. Le séparateur air/huile 24 comprend une paroi interne 24.5 formant avec la paroi circulaire 24.1 un bassin de décantation 24.6 pour l'huile séparée de l'air et s'écoulant le long de la paroi circulaire 24.1. Ce bassin est particulièrement intéressant en ce qu'il permet aux particules contenues dans l'huile de s'accumuler dans le bassin de décantation 24.6 tout en permettant à l'huile de s'écouler, par débordement, vers l'enceinte 26 du réservoir, via la sortie centrale d'huile 24.4.

Le dispositif 25 de détection de particules dans l'huile de lubrification comprend un conduit de dérivation 25.1 connecté de manière fluidique à une sortie 24.7 du bassin de décantation 24.6 de manière à former un débit d'huile réduit parallèle au débit d'huile principal depuis le séparateur air/huile 24 vers l'enceinte 26 du réservoir. Le conduit de dérivation 25.1 rejoint l'écoulement d'huile principal ou directement l'enceinte 26. Le dispositif 25 de détection de particules dans l'huile de lubrification comprend également un ou plusieurs détecteurs de particules 25.2 et 25.3. Chacun de ces détecteurs est couplé de manière opérationnelle au conduit de dérivation 25.1 de manière à détecter les éventuelles particules contenues dans l'huile circulant dans le conduit de dérivation 25.1.

Le ou les détecteurs de particules peuvent être de différents types. Un premier type peut être pour détecter les particules métalliques, comme par exemple les détecteurs commercialisés sous le nom Metallscan^{®}, notamment de la série MS1000, par la société Gastops^{®}, ou encore sous le nom QDM^{®} par la société Eaton^{®}. Un deuxième type peut être pour détecter les particules non-métalliques, comme par exemple des détecteurs optiques ou à vibrations.

Les détecteurs de particules 25.2 et 25.3 sont avantageusement reliés électriquement à une unité de contrôle et/ou d'évaluation 28 permettant de produire des informations structurées quant à la présence de particules dans l'huile, comme notamment la nature des particules (métalliques, non-métalliques), leur concentration et/ou quantité (par exemple en masse).

Le débit d'huile dans le conduit de dérivation 25.1 est plus faible que le débit principal depuis le séparateur air/huile 24 vers l'enceinte 26 du réservoir. Ce débit peut être produit par gravité et/ou au moyen d'une pompe (non représentée) disposé par exemple, de manière fluidique, dans le conduite de dérivation 25.1. Il peut s'agir d'une pompe à faible débit, comme par exemple une pompe de dosage.

Le débit réduit le long du conduit de dérivation 25.1 est particulièrement favorable à la détection de particules, qu'elles soient métalliques ou non métalliques. Une section réduite, par rapport à un conduit principal, du conduit de dérivation 25.1 et une vitesse de déplacement limitée au sein du conduit en question permet à chacun des détecteurs de particules d'être actifs au niveau détection sur la totalité ou quasi-totalité de la section du conduit de dérivation 25.1 et de détecter avec un plus grande fiabilité toute particule circulant dans le conduit de dérivation 25.1. La section de passage moyenne du conduit de dérivation 25.1 est avantageusement inférieure ou égale à 700mm², 600 mm² ou encore 500mm². La vitesse de déplacement de l'huile dans le conduit de dérivation 25.1 est avantageusement inférieure ou égale à 2m/s, 1m/s ou encore 0.5m/s.

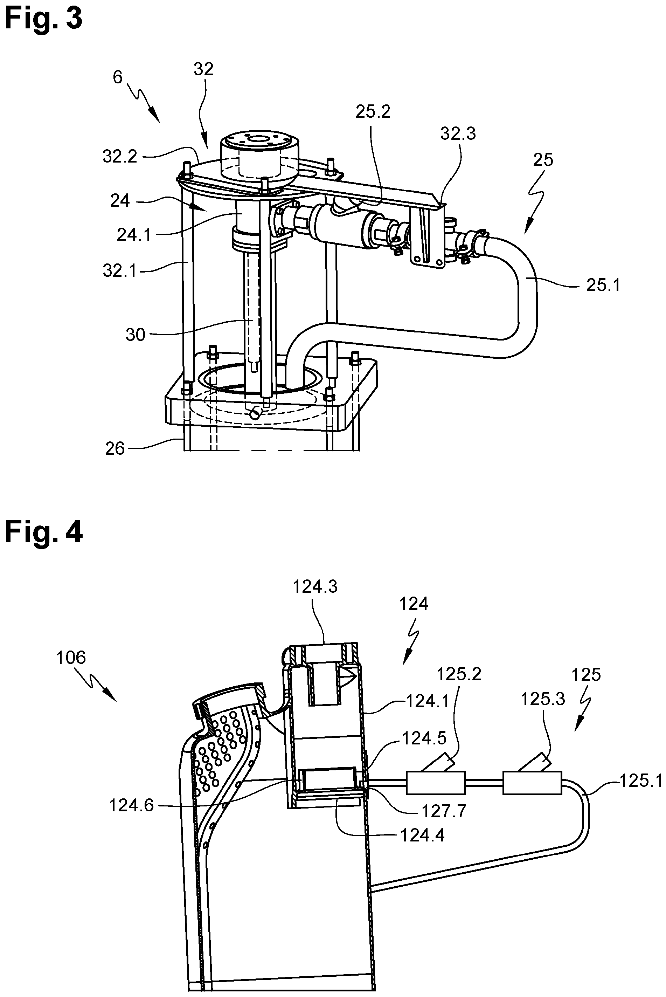

La figure 3 est une vue en perspective d'un séparateur air/huile avec un dispositif de détection de particules, tel que schématisé à la figure 2.

On peut observer que le séparateur air/huile 24 est rigidement fixé à l'enceinte 26 au moyen d'un support 32. Ce dernier comprend des tiges 32.1 rigidement fixées à une platine supérieure de l'enceinte 26. Aux extrémités distales de ces tiges est fixée une platine 32.2 du support 32. Le séparateur air/huile 24 est fixé à la platine 32.3 du support 32.

Le support 32 comprend aussi un bras 32.3 s'étendant essentiellement radialement par rapport à un axe longitudinal du réservoir 6, configuré pour supporter le conduit de dérivation 25.1 du dispositif de détection de particules 25. En l'occurrence le bras 32.3 s'étend depuis la platine 32.2. Il comprend une bride de fixation d'un raccord du conduit de dérivation 25.1. En l'occurrence, un seul détecteur de particules 25.2 est présent. Il est disposé entre la bride de fixation du bras 32.3 et la paroi circulaire 24.1 du séparateur air/huile 24.

On peut aussi observer le conduit 30 reliant la sortie centrale d'huile du séparateur air/huile 24 et l'enceinte 26. Il s'étend essentiellement longitudinalement en position centrale par apport à l'enceinte 26.

La figure 4 est une vue en coupe d'un séparateur air/huile avec un dispositif de détection de particules, tel que schématisé à la figure 2, intégré dans l'enceinte du réservoir d'huile de lubrification de turbomachine. Les numéros de références des figures 1 à 3 sont utilisés pour désigner les mêmes éléments, ces numéros étant toutefois majorés de 100. Il est par ailleurs fait référence à la description de ces éléments en relation avec les figures 1 à 3.

On peut observer que la paroi circulaire 124.1 du séparateur air/huile 124 est partiellement intégrée dans la paroi de l'enceinte 126 du réservoir 106. La sortie d'huile 124.4 débouche alors directement dans l'enceinte 126 sans nécessairement passer par une conduite ou tuyau.

On peut aussi observer que la paroi circulaire 124.1 est généralement cylindrique sans comporter de portion inférieure conique comme à la figure 2. Le fond de la cuve de décantation 124.6 est alors généralement plat et annulaire autour de la paroi interne 124.5 délimitant le bassin de décantation 124.6.

Le dispositif 125 de détection de particules dans l'huile de lubrification comprend, similairement à la figure 2, le conduit de dérivation 125.1 connecté de manière fluidique au bassin de décantation 124.6 de manière à former un débit d'huile réduit parallèle au débit d'huile principal depuis le séparateur air/huile 124 vers l'enceinte 126 du réservoir.

De manière générale, il est envisageable de prévoir plusieurs conduits de dérivation connectés de manière fluidique à un même circuit de lubrification, plus particulièrement à un même réservoir d'huile ou encore à un même séparateur air/huile. Le séparateur de particules comprend alors plusieurs sorties dont chacune est reliée à un des conduits de dérivation, respectivement. Les plusieurs sorties du séparateur de particules peuvent alors être configurées pour séparer différents types et/ou tailles de particules. Chaque conduit de dérivation peut alors être configuré pour spécifiquement détecter un de ces types et/ou une de ces tailles de particules. Dans le cas où le séparateur de particules est formé par un séparateur air/huile du type cyclonique avec une paroi de ruissellement d'huile, cette paroi peut comporter les plusieurs sorties à différents niveaux suivant la direction longitudinale de ladite paroi. Différents bassins de décantation peuvent alors être prévus sur la paroi en question, aux différents niveaux de manière à retenir et donc séparer les particules spécifiquement projetées contre la paroi entre ce niveau et le niveau adjacent supérieur.

## Revendications

1. Dispositif de détection (25 ; 125) de particules dans une huile de lubrification d'une turbomachine, notamment d'aéronef, comprenant :
- un séparateur des particules (24.1, 24.5, 24.6; 124.1, 124.5, 124.6) comportant une paroi circulaire (24.1 ; 124.1) ;
- un conduit de dérivation (25.1 ; 125.1) pour l'huile concentrant les particules, distinct du séparateur des particules (24.1, 24.5, 24.6 ; 124.1, 124.5, 124.6) et relié de manière fluidique à une sortie d'huile (24.7 ; 124.7) dudit séparateur des particules (24.1, 24.5, 24.6 ; 124.1, 124.5, 124.6) de manière à pouvoir former un débit d'huile réduit parallèle à un débit d'huile principal, concentrant les particules ; et
- au moins un détecteur de particules (25.2, 25.3 ; 125.2, 125.3) monté de manière opérationnelle sur le conduit de dérivation (25.1 ; 125.1) de manière à pouvoir détecter les particules dans ledit conduit de dérivation ;
**caractérisé en ce que** le séparateur des particules (24.1, 24.5, 24.6; 124.1, 124.5, 124.6) comprend une paroi interne (24.5 ; 124.5) formant avec la paroi circulaire (24.1 ; 124.1) un bassin de décantation de l'huile (24.6 ; 124.6), ledit bassin de décantation (24.3 ; 124.6) étant formé autour de la paroi interne (24.5 ; 124.5), la sortie d'huile (24.7 ; 124.7) étant une sortie fluidique dudit bassin de décantation (24.6 ; 124.6).

2. Dispositif de détection (25 ; 125) selon la revendication 1, dans lequel la paroi circulaire (24.1; 124.1) est une paroi de ruissellement pour l'huile vers le bassin de décantation.

3. Dispositif de détection (25 ; 125) selon la revendication 2, dans lequel la paroi circulaire (24.1; 124.1) forme un cyclone pour un mélange de l'huile avec de l'air.

4. Dispositif de détection (25 ; 125) selon l'une des revendications 1 à 3, dans lequel le séparateur de particules (24.1, 24.5, 24.6; 124.1, 124.5, 124.6) est formé dans un séparateur air/huile (24 ; 124).

5. Dispositif de détection (25 ; 125) selon l'une des revendications 1 à 3, et selon la revendication 4, dans lequel le séparateur air/huile (24 ; 124) est du type cyclonique avec une entrée pour l'huile chargée d'air (24.2 ; 124.2), une sortie d'air (24.3 ; 124.3) et une sortie d'huile déchargée d'air (24.4 ; 124.4), le bassin de décantation (24.6 ; 124.6) étant situé de manière fluidique entre l'entrée pour l'huile chargée d'air (24.2 ; 124.2) et la sortie d'huile déchargée d'air (24.4 ; 124.4).

6. Dispositif de détection (25 ; 125) selon l'une des revendications 1 à 5, dans lequel l'au moins un détecteur de particules (25.2, 25.3 ; 125.2, 125.3) comprend un détecteur, préférentiellement optique, apte à détecter des particules non ferromagnétiques.

7. Dispositif de détection (25 ; 125) selon l'une des revendications 1 à 6, dans lequel l'au moins un détecteur de particules (25.2, 25.3 ; 125.2, 125.3) comprend au moins un détecteur magnétique apte à détecter des particules ferromagnétiques.

8. Dispositif de détection (25 ; 125) selon l'une des revendications 1 à 7, dans lequel le conduit de dérivation (25.1 ; 125.1) est un premier conduit de dérivation et la sortie d'huile du séparateur de particules est une première sortie d'huile, ledit dispositif détection comprenant au moins un deuxième conduit de dérivation relié de manière fluidique à une deuxième sortie d'huile du séparateur des particules (24.1, 24.5, 24.6 ; 124.1, 124.5, 124.6), concentrant les particules, et au moins un de l'au moins un détecteur de particules est monté de manière opérationnelle sur le deuxième conduit de dérivation de manière à pouvoir détecter les particules dans ledit conduit de dérivation.

9. Dispositif de détection (25 ; 125) selon l'une des revendications 2 à 4, et selon la revendication 8, dans lequel le bassin de décantation de l'huile (24.6 ; 124.6) est un premier bassin de décantation, le séparateur des particules comprenant un deuxième bassin de décantation de l'huile, la deuxième sortie d'huile étant une sortie fluidique dudit deuxième bassin de décantation.

10. Réservoir d'huile de lubrification (6 ; 106) pour système de lubrification d'une turbomachine, notamment d'aéronef, comprenant :
- une enceinte (26 ; 126) pour l'huile de lubrification ;
- un dispositif de détection de particules (25 ; 125) dans l'huile de lubrification, disposé en amont de l'enceinte (26 ; 126) pour l'huile de lubrification ;
**caractérisé en ce que**
le dispositif de détection (25 ; 125) est selon l'une des revendications 1 à 9.

11. Réservoir d'huile de lubrification (6) selon la revendication 10, dans lequel le séparateur des particules (24.1, 24.5, 24.6) est à distance de l'enceinte (26), un conduit (30) reliant de manière fluidique ledit séparateur de particules (24.1, 24.5, 24.6) à la dite enceinte (26).

12. Réservoir d'huile de lubrification (6) selon la revendication 11, le dispositif de détection (25) est rigidement fixé à l'enceinte (26) par un support (32).

13. Réservoir d'huile de lubrification (106) selon la revendication 10, dans lequel le séparateur des particules (124.1, 124.5, 124.6) est intégré dans l'enceinte (126).

14. Réservoir d'huile de lubrification (106) selon l'une des revendications 10 à 13, dans lequel le conduit de dérivation (25.1 ; 125.1) rejoint l'écoulement d'huile principal depuis le séparateur de particules (24.1, 24.5, 24.6 ; 124.1, 124.5, 124.6) vers l'enceinte (26 ; 126), ou directement l'enceinte (26 ; 126).

15. Système de lubrification de turbomachine, notamment d'aéronef, comprenant :
- des conduites (8, 12, 18, 22) d'amenée et de retour d'huile de lubrification ;
- au moins une pompe de circulation (10, 20) de l'huile de lubrification dans les conduites (8, 12, 18, 22) ;
- un réservoir d'huile de lubrification (6) relié de manière fluidique aux conduites (8, 12, 18, 22) et à l'au moins une pompe de circulation (10, 20) ;
**caractérisé en ce que**
le réservoir d'huile de lubrification est selon l'une des revendications 10 à 14.

16. Turbomachine (2), notamment d'aéronef, comprenant un dispositif de détection (25) de particules dans une huile de lubrification, **caractérisé en ce que** ledit dispositif de détection est selon l'une des revendications 1 à 9.

## Patentansprüche

1. Erkennungsvorrichtung (25; 125) für Partikel im Schmieröl einer Turbomaschine, insbesondere eines Luftfahrzeugs, umfassend:
- einen Partikelabscheider (24.1, 24.5, 24.6; 124.1, 124.5, 124.6) mit einer kreisförmigen Wand (24.1; 124.1);
- eine Bypass-Leitung (25.1; 125.1) für das Öl, welches die Partikel konzentriert, getrennt vom Partikelabscheider (24.1, 24.5, 24.6; 124.1, 124.5, 124.6) und fluidisch mit einem Ölauslass (24.7; 124.7) des besagten Partikelabscheiders (24.1, 24.5, 24.6; 124.1, 124.5, 124.6) verbunden, um einen reduzierten Öldurchfluss parallel zu einem Haupt-Öldurchfluss zu bilden, der die Partikel konzentriert; und
- mindestens einen Partikeldetektor (25.2, 25.3; 125.2, 125.3), der funktional an der Bypass-Leitung (25.1; 125.1) montiert ist, um die Partikel in besagter Bypass-Leitung zu erkennen;
**dadurch gekennzeichnet, dass** der Partikelabscheider (24.1, 24.5, 24.6; 124.1, 124.5, 124.6) eine innere Wand (24.5; 124.5) beinhaltet, die zusammen mit der kreisförmigen Wand (24.1; 124.1) ein Absetzbassin (24.6; 124.6) für das Öl bildet, wobei besagtes Absetzbassin (24.3; 124.6) um die innere Wand (24.5; 124.5) gebildet wird, wobei der Ölauslass (24.7; 124.7) ein fluidischer Auslass des besagten Absetzbeckens (24.6; 124.6) ist.

2. Erkennungsvorrichtung (25; 125) nach Anspruch 1, wobei die kreisförmige Wand (24.1; 124.1) eine Abflusswand für das Öl in das Absetzbassin ist.

3. Erkennungsvorrichtung (25; 125) nach Anspruch 2, wobei die kreisförmige Wand (24.1; 124.1) einen Zyklon für ein Gemisch des Öls mit Luft bildet.

4. Erkennungsvorrichtung (25; 125) nach einem der vorhergehenden Ansprüche 1 bis 3, wobei der Partikelabscheider (24.1, 24.5, 24.6; 124.1, 124.5, 124.6) in einem Luft/Öl-Abscheider (24; 124) integriert ist.

5. Erkennungsvorrichtung (25; 125) nach einem der vorhergehenden Ansprüche 1 bis 4, wobei der Luft/Öl-Abscheider (24; 124) zyklonartig ist mit einem Einlass für das ölbeladene Luftgemisch (24.2; 124.2), einem Luftauslass (24.3; 124.3) und einem Ölauslass für entlüftetes Öl (24.4; 124.4), wobei das Absetzbassin (24.6; 124.6) fluidisch zwischen dem Einlass für das ölbeladene Luftgemisch (24.2; 124.2) und dem Ölauslass für entlüftetes Öl (24.4; 124.4) liegt.

6. Erkennungsvorrichtung (25; 125) nach einem der vorhergehenden Ansprüche 1 bis 5, wobei mindestens ein Partikeldetektor (25.2, 25.3; 125.2, 125.3) einen Detektor, vorzugsweise optisch, umfasst, der in der Lage ist, nicht ferromagnetische Partikel zu erkennen.

7. Erkennungsvorrichtung (25; 125) nach einem der vorhergehenden Ansprüche 1 bis 6, wobei mindestens ein Partikeldetektor (25.2, 25.3; 125.2, 125.3) mindestens einen magnetischen Detektor umfasst, der in der Lage ist, ferromagnetische Partikel zu erkennen.

8. Erkennungsvorrichtung (25; 125) nach einem der vorhergehenden Ansprüche 1 bis 7, wobei die Bypass-Leitung (25.1; 125.1) eine erste Bypass-Leitung ist und der Ölauslass des Partikelabscheiders ein erster Ölauslass ist, wobei besagte Erkennungsvorrichtung mindestens eine zweite Bypass-Leitung umfasst, die fluidisch mit einem zweiten Ölauslass des Partikelabscheiders (24.1, 24.5, 24.6; 124.1, 124.5, 124.6), die die Partikel konzentriert, verbunden ist, und mindestens einer der mindestens einen Partikeldetektor ist funktional an der zweiten Bypass-Leitung montiert, um die Partikel in besagter Bypass-Leitung zu erkennen.

9. Erkennungsvorrichtung (25; 125) nach einem der vorhergehenden Ansprüche 2 bis 4, und nach Anspruch 8, wobei das Absetzbassin für das Öl (24.6; 124.6) ein erstes Absetzbassin ist, wobei der Partikelabscheider ein zweites Absetzbassin für das Öl umfasst und der zweite Ölauslass ein fluidischer Auslass des besagten zweiten Absetzbeckens ist.

10. Schmieröltank (6; 106) für das Schmierölsystem einer Turbomaschine, insbesondere eines Luftfahrzeugs, umfassend:
- ein Gehäuse (26; 126) für das Schmieröl;
- eine Erkennungsvorrichtung für Partikel (25; 125) im Schmieröl, die stromaufwärts des Gehäuses (26; 126) für das Schmieröl angeordnet ist;
**dadurch gekennzeichnet, dass** die Erkennungsvorrichtung (25; 125) nach einem der vorhergehenden Ansprüche 1 bis 9 ist.

11. Schmieröltank (6) nach Anspruch 10, wobei der Partikelabscheider (24.1, 24.5, 24.6) sich entfernt von dem Gehäuse (26) befindet, wobei eine Leitung (30) besagten Partikelabscheider (24.1, 24.5, 24.6) fluidisch mit dem besagten Gehäuse (26) verbindet.

12. Schmieröltank (6) nach Anspruch 11, wobei die Erkennungsvorrichtung (25) fest mit dem Gehäuse (26) durch eine Halterung (32) verbunden ist.

13. Schmieröltank (106) nach Anspruch 10, wobei der Partikelabscheider (124.1, 124.5, 124.6) in das Gehäuse (126) integriert ist.

14. Schmieröltank (106) nach einem der vorhergehenden Ansprüche 10 bis 13, wobei die Bypass-Leitung (25.1; 125.1) den Haupt-Ölfluss vom Partikelabscheider (24.1, 24.5, 24.6; 124.1, 124.5, 124.6) zum Gehäuse (26; 126) oder direkt zum Gehäuse (26; 126) verbindet.

15. Schmierölsystem einer Turbomaschine, insbesondere eines Luftfahrzeugs, umfassend:
- Leitungen (8, 12, 18, 22) zur Zufuhr und Rückführung von Schmieröl;
- mindestens eine Umlaufpumpe (10, 20) für das Schmieröl in den Leitungen (8, 12, 18, 22);
- einen Schmieröltank (6), der fluidisch mit den Leitungen (8, 12, 18, 22) und mindestens einer Umlaufpumpe (10, 20) verbunden ist;
**dadurch gekennzeichnet, dass**
der Schmieröltank gemäß einem der Ansprüche 10 bis 14 ist.

16. Turbomaschine (2), insbesondere eines Luftfahrzeugs, umfassend eine Erkennungsvorrichtung (25) für Partikel im Schmieröl, **dadurch gekennzeichnet, dass** besagte Erkennungsvorrichtung gemäß einem der Ansprüche 1 bis 9 ist.

## Claims

1. A detection device (25; 125) for particles in lubrication oil of a turbomachine, notably an aircraft engine, comprising:
- a particle separator (24.1, 24.5, 24.6; 124.1, 124.5, 124.6) comprising a circular wall (24.1; 124.1);
- a bypass conduit (25.1; 125.1) for oil concentrating the particles, distinct from the particle separator (24.1, 24.5, 24.6; 124.1, 124.5, 124.6) and fluidically connected to an oil outlet (24.7; 124.7) of said particle separator (24.1, 24.5, 24.6; 124.1, 124.5, 124.6) so as to form a reduced oil flow parallel to a main oil flow, concentrating the particles; and
- at least one particle detector (25.2, 25.3; 125.2, 125.3) operationally mounted on the bypass conduit (25.1; 125.1) so as to detect particles in said bypass conduit;
**characterized in that** the particle separator (24.1, 24.5, 24.6; 124.1, 124.5, 124.6) comprises an internal wall (24.5; 124.5) forming with the circular wall (24.1; 124.1) a settling basin for the oil (24.6; 124.6), said settling basin (24.3; 124.6) being formed around the internal wall (24.5; 124.5), the oil outlet (24.7; 124.7) being a fluid outlet of said settling basin (24.6; 124.6).

2. The detection device (25; 125) according to claim 1, wherein the circular wall (24.1; 124.1) is a runoff wall for the oil towards the settling basin.

3. The detection device (25; 125) according to claim 2, wherein the circular wall (24.1; 124.1) forms a cyclone for a mixture of the oil with air.

4. The detection device (25; 125) according to any one of claims 1 to 3, wherein the particle separator (24.1, 24.5, 24.6; 124.1, 124.5, 124.6) is formed in an air/oil separator (24; 124).

5. The detection device (25; 125) according to any one of claims 1 to 3, and according to claim 4, wherein the air/oil separator (24; 124) is of the cyclonic type with an inlet for oil loaded with air (24.2; 124.2), an air outlet (24.3; 124.3), and an oil outlet discharged of air (24.4; 124.4), the settling basin (24.6; 124.6) being fluidically located between the inlet for oil loaded with air (24.2; 124.2) and the oil outlet discharged of air (24.4; 124.4).

6. The detection device (25; 125) according to any one of claims 1 to 5, wherein the at least one particle detector (25.2, 25.3; 125.2, 125.3) comprises a detector, preferably optical, capable of detecting nonferromagnetic particles.

7. The detection device (25; 125) according to any one of claims 1 to 6, wherein the at least one particle detector (25.2, 25.3; 125.2, 125.3) comprises at least one magnetic detector capable of detecting ferromagnetic particles.

8. The detection device (25; 125) according to any one of claims 1 to 7, wherein the bypass conduit (25.1; 125.1) is a first bypass conduit and the oil outlet of the particle separator is a first oil outlet, said detection device comprising at least one second bypass conduit fluidically connected to a second oil outlet of the particle separator (24.1, 24.5, 24.6; 124.1, 124.5, 124.6), concentrating the particles, and at least one of the at least one particle detector is operationally mounted on the second bypass conduit so as to detect particles in said bypass conduit.

9. The detection device (25; 125) according to any one of claims 2 to 4, and according to claim 8, wherein the oil settling basin (24.6; 124.6) is a first settling basin, the particle separator comprising a second oil settling basin, the second oil outlet being a fluid outlet of said second settling basin.

10. A lubrication oil tank (6; 106) for a lubrication system of a turbomachine, notably an aircraft engine, comprising:
- an enclosure (26; 126) for the lubrication oil;
- a particle detection device (25; 125) in the lubrication oil, disposed upstream of the enclosure (26; 126) for the lubrication oil;
**characterized in that** the detection device (25; 125) is according to any one of claims 1 to 9.

11. The lubrication oil tank (6) according to claim 10, wherein the particle separator (24.1, 24.5, 24.6) is at a distance from the enclosure (26), a conduit (30) fluidically connecting said particle separator (24.1, 24.5, 24.6) to said enclosure (26).

12. The lubrication oil tank (6) according to claim 11, wherein the detection device (25) is rigidly attached to the enclosure (26) by a support (32).

13. A lubrication oil tank (106) according to claim 10, wherein the particle separator (124.1, 124.5, 124.6) is integrated into the enclosure (126).

14. The lubrication oil tank (106) according to any one of claims 10 to 13, wherein the bypass conduit (25.1; 125.1) joins the main oil flow from the particle separator (24.1, 24.5, 24.6; 124.1, 124.5, 124.6) to the enclosure (26; 126), or directly to the enclosure (26; 126).

15. A lubrication system for a turbomachine, notably an aircraft engine, comprising:
- supply and return lubrication oil conduits (8, 12, 18, 22);
- at least one pump (10, 20) for circulating the lubrication oil in the conduits (8, 12, 18, 22);
- a lubrication oil tank (6) fluidically connected to the conduits (8, 12, 18, 22) and to the at least one circulation pump (10, 20);
**characterized in that**
the lubrication oil tank is according to one of claims 10 to 14.

16. Turbomachine (2), notably an aircraft engine, comprising a detection device (25) for particles in lubrication oil, **characterized in that** said detection device is according to one of claims 1 to 9.
